(19) Europäisches Patentamt
European Patent Office
Office européen des brevets

(11) EP 2 900 649 B1

(12) **FASCICULE DE BREVET EUROPEEN**

(45) Date de publication et mention de la délivrance du brevet:
**04.07.2018 Bulletin 2018/27**

(21) Numéro de dépôt: **13779317.0**

(22) Date de dépôt: **26.09.2013**

(51) Int Cl.:
***C07D 307/46*** (2006.01) ***C07D 307/68*** (2006.01)

(86) Numéro de dépôt international:
**PCT/FR2013/052273**

(87) Numéro de publication internationale:
**WO 2014/049276 (03.04.2014 Gazette 2014/14)**

(54) **PROCEDE DE SYNTHESE DE L'ACIDE 2,5-FURANE DICARBOXYLIQUE A PARTIR D'UNE COMPOSITION CONTENANT DU FURANE-2,5-DIALDEHYDE**

VERFAHREN ZUR SYNTHESE VON 2,5-FURANDICARBONSÄURE AUS EINER ZUSAMMENSETZUNG MIT FURAN-2,5-DIALDEHYD

METHOD FOR SYNTHESISING 2,5-FURANDICARBOXYLIC ACID FROM A COMPOSITION CONTAINING FURAN-2,5-DIALDEHYDE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.09.2012 FR 1259103**

(43) Date de publication de la demande:
**05.08.2015 Bulletin 2015/32**

(73) Titulaire: **Roquette Frères**
**62136 Lestrem (FR)**

(72) Inventeurs:
- **IBERT, Mathias**
  **F-59930 La Chapelle d'Armentieres (FR)**
- **CHAMBON, Flora**
  **F-59700 Marc en Baroeul (FR)**
- **DAMBRINE, Laurent**
  **F-62114 Sains en Gohelle (FR)**

(74) Mandataire: **Cabinet Plasseraud**
**66, rue de la Chaussée d'Antin**
**75440 Paris Cedex 09 (FR)**

(56) Documents cités:
**WO-A1-2012/004069 WO-A1-2013/093322**

- **HAJJ EL T ET AL: "SYNTHESE DE L'HYDROXYMETHYL-5 FURANNE CARBOXALDEHYDE-2 ET DE SES DERIVES PAR TRAITEMENT ACIDE DE SUCRES SUR RESINES ECHANGEUSES D'IONS", BULLETIN DE LA SOCIETE CHIMIQUE DE FRANCE, SOCIETE FRANCAISE DE CHIMIE. PARIS, FRANCE, vol. 5, 1 janvier 1987 (1987-01-01), pages 855-860, XP009002108, ISSN: 0037-8968**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 900 649 B1

## Description

La présente invention concerne un procédé de synthèse de l'acide 2,5-furane dicarboxylique (FDCA) à partir d'une composition contenant du furane-2,5-dialdéhyde (DFF). Un tel procédé est facilement industrialisable : il est très simple à mettre en oeuvre, les réactifs utilisés sont facilement disponibles, les conditions expérimentales sur lesquelles il repose sont très «douces», à savoir faibles températures et pression atmosphérique. De plus, il ne conduit pas à la formation de sels qui nécessitent d'être traités en aval. Enfin, la quantité de catalyseur engagée par rapport au produit initial est particulièrement faible, ce qui représente un avantage économique indéniable eu égard au prix dudit catalyseur.

La présente invention concerne donc un procédé de fabrication de l'acide 2,5-furane dicarboxylique, désigné sous le terme FDCA et répondant à la formule chimique suivante :

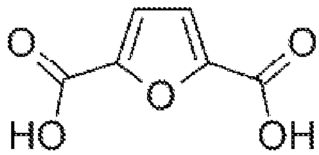

Le FDCA est utilisé en pharmacologie, où son ester diéthylique a fait preuve d'une forte activité anesthésique, comparable à celle de la cocaïne. C'est également un agent chélatant très puissant, permettant de complexer de nombreux ions comme $Ca^{2+}$, $Cu^{2+}$ et $Pb^{2+}$. En médecine, il est utilisé pour traiter les calculs rénaux, mais aussi dans la préparation de transplants ayant des propriétés biologiques analogues à celles des organes naturels, et qui sont caractérisés par une absence de rejet après la greffe.

Mais le FDCA est avant tout un monomère de base entrant dans la fabrication de nombreux polymères, tels que les polyesters, les polyamides ou encore les polyuréthanes, permettant notamment d'en améliorer les propriétés mécaniques. Dans les polyesters, il est susceptible d'être utilisé en remplacement des phthalates. Au regard d'une telle possibilité, le FDCA a été classé parmi les 12 matières premières issues du végétal offrant le plus grand potentiel industriel (Werpy, T., Peterson, G., 2004. Top Value Added Chemicals from Biomass, vol. 1, pp. 26-28).

Il existe à ce jour 4 voies de synthèse du FDCA. La première repose sur la déshydratation de dérivés d'hexoses. Une telle réaction n'est possible que sous des conditions opératoires très sévères : l'utilisation de solutions acides hautement concentrées, une température supérieure à 120 °C et des temps de réaction de l'ordre de la demi-journée. Le document FR 2 723 945 en est une illustration.

Une autre route consiste à réaliser la conversion catalytique de dérivés furaniques. Le furfural peut par exemple être oxydé au moyen d'acide nitrique en acide 2-furoïque, ce dernier étant ensuite converti en son ester méthylique. L'ester est transformé par chlorométhylation en 5-chlorométhylfuroate. Ce dernier est oxydé à nouveau par l'acide nitrique pour former du 2,5-diméthyl furandicarboxylate qui, après hydrolyse alcaline, conduit au FDCA avec un rendement de l'ordre de 50 % seulement. Parallèlement, il a été démontré que le 2-fuorate de potassium, chauffé à 300 °C sous atmosphère d'azote, conduisait à du di-potassium 2,5-furandicarboxylate (Andrisano, R.; Angeloni, A.S. Ann. Chim. (Rome) 1963, 53, 1658)

Une troisième possibilité, sans doute aujourd'hui la plus utilisée industriellement, consiste à oxyder des dérivés de furane disubstitués en position 2 et 5, dont le 5-hydroxyméthyl furaldéhyde (5-HMF) et le furane-2,5-dialdéhyde ou 2,5-diformylfurane (DFF). Ainsi, le brevet FR 2 669 634 décrit un procédé d'oxydation catalytique du 5-HMF conduisant à la synthèse de FDCA. Ce procédé consiste à oxyder le 5-HMF en milieu aqueux alcalin, sous courant d'oxygène, en présence d'un catalyseur à base de platine activé au plomb supporté sur charbon. Un tel procédé présente l'inconvénient de générer de grosses quantités de sels.

De manière similaire, le brevet WO 2012/017052 décrit la synthèse de FDCA par oxydation de 5-HMF en milieu basique en présence d'un catalyseur platine supporté sur charbon, à 100 °C et sous une pression de 5 bars, avec formation d'une quantité de sels importante.

Le DFF peut également servir de produit de départ, comme divulgué dans le brevet US 2007/0232815. On y décrit la synthèse du FDCA par oxydation du DFF en milieu aqueux alcalin, en présence d'oxygène, le catalyseur étant notamment le permanganate de potassium. Néanmoins, ce procédé qui aboutit à un rendement de 89 % fait état d'un ratio catalytique très important par rapport au DFF, égal à 195 % (ratio en poids) dans son exemple 4.

Dans cette lignée, le document « Synthèse de l'hydroxyméthyl-5 furanne carboxaldehyde-2 et de ses dérivés par traitement acide de sucres sur résines échangeuses d'ions » (Bulletin de la Société Chimique de France, Soc. Fr. de Chim., vol 5, 1er janvier 1987, pp. 855-860) divulgue en page 859 la réaction du DFF en FDCA catalysée par l'oxyde d'argent dans une solution aqueuse de soude. Le rendement de la réaction est de 80 %, avec un ratio en poids catalyseur par rapport au DFF égal à 222,5 %.

Cet article de M. Hajj et Co décrit aussi à la page 860 colonne 1 l'oxydation du HMF en présence d'un large excès de permanganate de potassium en tant qu'oxydant et de pyridine en tant que solvant. Le milieu réactionnel est

ensuite concentré, repris à l'eau, filtré pour éliminer le bioxyde de manganèse, concentré à nouveau puis acidifié. Enfin le produit réactionnel obtenu est recristallisé. Selon ce procédé le FDCA est obtenu avec un rendement de 70%.

Cet article ne décrit donc nullement l'oxydation de DFF à un pH inférieur à 4 en présence d'acide péracétique, et dans un solvant choisi parmi l'eau, l'acide acétique et leurs mélanges, et permettant de manière simple et rapide l'obtention directe de FDCA et non d'un sel, sans traitement ultérieur (filtration, acidification etc.) et ceci avec un très bon rendement.

Enfin, toujours dans cette catégorie, le document « Benzene-, Pyrrole-, and Furan-containing diametrically strapped calix[4]pyrroles - An experimental and theoretical study of hydrogen-bonding effects in chloride anion récognition » (Angewandte Chemie International Edition, vol 47, n° 27, 23 juin 2008, pp. 5038-5042) décrit la synthèse de FDCA à partir de DFF (schéma 1) en présence de KMnO4 dans une solution aqueuse de soude. Le rendement est de 60 %, sans qu'aucun ratio catalytique (ratio en poids catalyseur/DFF) ne soit mentionné.

Il est important de noter que ces 3 documents conduisent en fait à la synthèse de sels de FDCA, et non de FDCA comme tel, puisque les synthèses sont toutes conduites en milieu basique (présence d'une solution aqueuse de soude).

Enfin, il existe un quatrième type de procédé, qui repose sur la conversion enzymatique du 5-HMF en FDCA en présence d'oxygène moléculaire, et à partir d'une 5-HMF/furfural oxydoréductase, qui a été isolée à partir de la bactérie Cupriavidus basilensis HMF14 (F. Koopman, N. Wierckx, J.H. de Winde and H.J. Ruijssenaars. Proc. Nat. Acad. Sci. USA. 2010, 107: 4919-4924). Ce procédé permet de travailler à pression et température ambiante, dans l'eau, et offre un rendement de l'ordre de 97 % pour une composition finale présentant une teneur en FDCA supérieure à 99 %. Néanmoins, l'enzyme utilisée n'est produite aujourd'hui qu'à toute petite échelle, ce qui n'est pas compatible avec les exigences d'un procédé industriel. De plus, ce procédé est générateur d'une quantité de sels non négligeable.

En résumé, les procédés de synthèse de FDCA selon l'art antérieur présentent les inconvénients d'opérer sous des conditions trop sévères de température et / ou de pression, de générer d'importantes quantités de sels qu'il faut traiter ultérieurement, d'offrir des rendements médiocres, d'utiliser un ratio catalytique important c'est-à-dire de consommer trop de catalyseur et de n'aboutir qu'à des rendements très médiocres, ou de s'appuyer sur une enzyme très particulière dont l'approvisionnement n'est aujourd'hui pas sécurisée en vue d'une production industrielle.

Poursuivant des recherches pour pallier de tels inconvénients, la Demanderesse est parvenue à mettre au point un nouveau procédé de fabrication de FDCA, à partir de l'oxydation de DFF, dans des conditions de température et de pression particulièrement douces (température inférieure à 100 °C, et pression atmosphérique). En outre, ce procédé peut être conduit dans l'eau, avec des ratios catalytiques très avantageux moins d'1 mole de catalyseur par mole de DFF). De plus, il n'y a pas formation de sels, ce qui évite tout problème de retraitement ou traitement ultérieur.

On parvient ainsi à produire du FDCA avec un taux de conversion de 100 % et une teneur en produit final supérieure à 70 % en poids, préférentiellement à 80 %, très préférentiellement à 90 %, et de manière extrêmement préférentielle supérieure à 99 %, de même qu'une sélectivité en FDCA supérieure à 70 %, préférentiellement à 80 %, très préférentiellement à 90 %. On aboutit alors à des rendements pour la plupart supérieurs à 80 % en poids de FDCA ainsi formés.

En outre, il n'est pas nécessaire de partir d'une composition présentant une haute teneur en DFF pour obtenir une grande sélectivité en FDCA (> 75 %). En effet, selon une variante particulière, un mélange DFF / FFCA (acide 5-furaldéhyde-2-carboxylique) issu de l'oxydation du 5-HMF (selon la technologie telle que brevetée par la Demanderesse dans le document portant le numéro de dépôt FR 11 62343) peut être utilisé comme réactif de départ.

Comme déjà expliqué, le procédé objet de la présente invention permet d'obtenir directement le FDCA et non un de ses sels. Ce procédé est notamment caractérisé en ce qu'il a lieu en milieu acide (pH < 7).

Dans toute la présente demande, on utilise les termes de sélectivité (S), de conversion (C) et de rendement (R) en référence aux définitions suivantes :

$$C\ (\%\ \text{molaire}) = ((\text{quantité de DFF transformée}) \times 100)\ /\ \text{quantité de DFF initiale}$$

$$S\ (\%\ \text{molaire}) = ((\text{quantité de FDCA formée}) \times 100)\ /\ \text{quantité de DFF transformée}$$

$$R\ (\%\ \text{molaire}) = S \times C\ /100 = ((\text{quantité de FDCA formée}) \times 100)\ /\ \text{quantité de DFF initiale}$$

Aussi, un premier objet de la présente invention consiste en un procédé de synthèse de FDCA à pH inférieur à 4, selon la revendication 1.

De manière tout à fait surprenante, la présente invention permet non seulement d'obtenir du FDCA directement,

avec des taux de conversion et des rendements très élevés, mais ceci en diminuant de manière extrêmement importante les quantités de catalyseur mis en oeuvre. Lorsqu'on met en perspective de ce résultat le coût de ces catalyseurs qui sont en général des produits issus de synthèses complexes, et leur dangerosité vis-à-vis des êtres humains mais aussi de l'environnement, on comprend tout l'intérêt du procédé objet de la présente invention.

Le réactif de départ est donc une composition contenant du DFF. A ce titre, il peut s'agir de DFF seul ou en mélange avec au moins un autre constituant. Avantageusement, lorsque le DFF n'est pas seul présent dans la composition, celle-ci consistera en une composition ou mélange DFF / FFCA en toutes proportions. De manière particulièrement avantageuse, ce mélange est issu de l'oxydation du 5-HMF selon le procédé objet de l'invention brevetée par la Demanderesse sous le numéro FR 11 62343.

Le procédé objet du brevet FR 11 62343 précité est caractérisé en ce qu'il comprend une étape d'oxydation en présence d'au moins un acide organique, d'un radical nitroxyl, d'une source en oxygène et d'un agent de transfert d'oxygène. Le radical nitroxyl est notamment choisi parmi les (2,2,6,6-tétraméthylpiperidin-1-yl)oxyl, encore dénommés TEMPO. En accord avec cette variante, le procédé selon l'invention présente alors l'avantage de synthétiser du FDCA en un procédé dit « one-pot », à partir de 5-HMF : par oxydation de 5-HMF en un mélange DFF / FFCA selon la méthode décrite dans le brevet FR 11 62343 sans l'étape de purification, puis oxydation de ce mélange en FDCA selon la présente invention.

Le procédé objet de la présente invention est donc caractérisé en ce que la synthèse de FDCA résultant de la mise en contact des composés précités, est réalisée à un pH inférieur à 4, très préférentiellement inférieur à 1. Le pH est ajusté par tous les moyens bien connus de l'homme du métier, notamment par adjonction d'un acide dans le milieu à acidifier.

Le solvant protique mis en oeuvre dans la présente invention au niveau de la synthèse de FDCA est choisi parmi l'eau et l'acide acétique et leurs mélanges. On évite ainsi la formation de sels qui nécessitent des moyens de traitement ultérieur, comme dans les techniques d'oxydation des dérivés de furane disubstitués en position 2 et 5.

La source en oxydant est l'acide peracétique. La Demanderesse a constaté que, lorsque l'acide peracétique était mis en oeuvre, il convenait de l'introduire goutte à goutte plutôt qu'en tout début de réaction, de manière à améliorer la sélectivité en FDCA.

Le catalyseur d'oxydation est $KMnO_4$. En outre, il n'est pas nécessaire de recourir à un co-catalyseur.

La réaction d'oxydation a lieu à une température comprise entre 3°C et la température de reflux du solvant (chauffage jusqu'à ébullition du solvant), préférentiellement entre 10 °C et la température de reflux du solvant (chauffage jusqu'à ébullition du solvant), et encore plus préférentiellement à une température comprise entre 40 °C et 60 °C. Elle peut éventuellement se dérouler sous pression, mais est réalisée avantageusement à pression atmosphérique. De telles conditions sont particulièrement « douces » et faciles à mettre en oeuvre, notamment par rapport à celles décrites dans l'art antérieur au sujet de la conversion catalytique de dérivés furaniques.

Le temps de contact entre les différents constituants a), b), c) et d) est compris entre 1 minute et 8 heures, préférentiellement entre 15 minutes et 2 heures. Il s'agit de temps de réaction relativement courts, notamment par rapport à ceux envisagés dans le document FR 2 723 945.

Le nombre d'équivalent molaire en oxydant par rapport au DFF est compris entre 0,1 et 10, plus préférentiellement entre 2,5 et 5.

Pour le reste, le procédé d'oxydation de la présente invention peut être réalisé dans tous types de réacteurs batch ou lit fixe, sous pression ou non, préférentiellement non. Le type de réacteur batch peut être un ballon en pyrex à fond plat, équipé d'un barreau d'agitation magnétique. Préférentiellement, le réacteur est en outre muni d'un système d'introduction goutte à goutte de la source en oxydant. Le réacteur peut en outre comprendre un système de refroidissement et également un système de mesure et de régulation de la température.

Au cours du procédé d'oxydation, le milieu réactionnel est maintenu sous agitation. La vitesse d'agitation est préférentiellement réglée entre 100 et 500 tours / minute, plus préférentiellement entre 100 et 250 tours / minute.

Un autre avantage relatif au procédé selon la présente invention réside dans la récupération du FDCA, particulièrement facile, puisque le FDCA en question est précipité au cours de sa formation ; il peut donc être récupéré, notamment à partir de moyens physiques, soit en fin de réaction, soit avantageusement au cours de la réaction c'est-à-dire qu'il est soutiré en continu pendant ladite réaction. Cette récupération aisée autorise donc un recyclage de l'oxydant et du catalyseur en excès ainsi que du solvant.

Les exemples qui suivent permettent de mieux appréhender la présente invention, sans toutefois en limiter la portée.

## EXEMPLES

Les tableaux suivants 1 à 3 répertorient les conditions expérimentales de différentes synthèses qui ont été réalisées selon les mêmes protocoles que ceux décrits ci-après :
Dans cette expérience, qui correspond à l'essai n° 3, on met en oeuvre :

- une composition contenant du DFF (95,1 % en poids en DFF et 4,9 % en poids de FFCA) : 4 g
- $KMnO_4$ (solution à 0.02 mol/L) : 2,53 g
- eau déminéralisée : 36 ml
- acide peracétique (solution à 38%) : 36g

Conditions opératoires :

Température de réaction : 50°C
Durée de réaction : 2h00 (introduction acide peracétique) + 3h00 (temps de contact)

Dans un ballon monocol à fond plat on introduit 4g d'une composition contenant du DFF et 2,53 g d'une solution de $KMnO_4$ à 0,02 mol / L, puis 36 g d'eau déminéralisée. Les ratios s'établissent de façon à avoir 0,2 % en poids de $KMnO_4$ par rapport au poids de DFF, ainsi qu'un équivalent molaire en oxydant par rapport au DFF de 5.

Le ballon est placé sous agitation magnétique dans un bain marie de façon à atteindre une température de réaction de 50°C. Sous agitation magnétique, 36 g d'une solution d'acide peracétique à 38 % en volume est ajoutée goutte à goutte à un débit de 0,3 g / min (soit environ 2h00 d'introduction goutte à goutte), menant à une acidification progressive du milieu réactionnel. A la fin de l'ajout d'acide peracétique, le milieu réactionnel est laissé sous agitation pendant encore 3h00. Le milieu réactionnel présente alors un pH inférieur à 0,5. Ensuite l'agitation magnétique est arrêtée et le milieu réactionnel analysé par chromatographie en phase gazeuse.

On détermine alors le taux de conversion ainsi que la sélectivité de la réaction à l'égard du FDCA.

Description essai n°17

Dans cette expérience qui correspond à l'essai n°17, on met en oeuvre :

- une composition contenant du DFF (93.4% poids de DFF) : 1.2g
- Acide perchlorique $HClO_4$ : 1.15g
- Méthanol : 46g
- $FeCl_3$ : 0.11g, soit 9% pds/composition contenant du DFF
- Peroxyde d'hydrogène (solution à 30%) : 8.6g

Conditions opératoires :

Température de réaction : 5°C
Durée de réaction : 10 minutes (introduction peroxyde d'hydrogène) + 4h00 (temps de contact)

Dans un ballon monocol à fond plat on introduit 1.2 g d'une composition contenant du DFF, 1,15 g d'acide perchlorique, 0,11 g de $FeCl_3$ puis 46 g de méthanol. Les ratios s'établissent de façon à avoir 9% pds de $FeCl_3$ par rapport au DFF, ainsi qu'un équivalent molaire en oxydant par rapport au DFF de 7,8.

Le ballon est placé sous agitation magnétique, puis le mélange réactionnel est amené à une température de réaction de 5°C. Sous agitation magnétique, 8,6 g d'une solution de peroxyde d'hydrogène à 30% en volume sont ajoutés goutte à goutte sur une durée totale de 10 minutes. A la fin de l'ajout de peroxyde d'hydrogène, le milieu réactionnel est laissé sous agitation pendant 4h00. Ensuite, la température de réaction est ramenée à température ambiante, puis l'agitation magnétique est arrêtée. Le milieu réactionnel est ensuite analysé par chromatographie en phase gazeuse.

On détermine alors le taux de conversion ainsi que la sélectivité de la réaction à l'égard du FDCA.

A noter que l'ensemble des essais a lieu à un pH compris entre 0 et 3, et conduisent à l'obtention de FDCA directement, et non à l'un de ses sels.

**Tableau 1**

| Essai n° | Masse composition contenant du DFF (g) | Solvant (masse g) |
|---|---|---|
| 1 | 4 | eau (36) |
| 2 | 4 | eau (36) |
| 3 | 4 | eau (36) |
| 4 | 2 | acide acétique (18) |
| 5 | 2 | acide acétique /eau (12/6) |

(suite)

| Essai n° | Masse composition contenant du DFF (g) | Solvant (masse g) |
|---|---|---|
| 6 | 2 | eau (18) |
| 7 | 2 | eau (18) |
| 8 | 2 | acide acétique (18) |
| 9 | 2 | eau (18) |
| 10 | 2 | eau (18) |
| 11 | 2 | eau (18) |
| 12 | 2 | eau (18) |
| 13 | 2 | eau (18) |
| 14 | 2 | eau (18) |
| 15 | 2 | eau (18) |
| 16 | 2 | eau (18) |
| 17 | 1,2 | MeOH/$HClO_4$ (46/1,15) |
| 18 | 1,2 | MeOH/$HClO_4$ (46/1,15) |

**Tableau 2**

| Essai n° | Source en oxydant | | | |
|---|---|---|---|---|
| | oxydant | masse (g) | Nombre d'équivalent molaire en oxydant par rapport au DFF | Durée introduction oxydant |
| 1 | acide peracétique (38%) | 32,1 | 5 | 3h00 |
| 2 | acideperacétique (38%) | 16,1 | 2,5 | 1h30 |
| 3 | acide peracétique (38%) | 36 | 5.6 | 3h00 |
| 4 | acideperacétique (38%) | 16 | 5 | 2h00 |
| 5 | Acide peracétique (38%) | 16 | 5 | 2h00 |
| 6 | Bactipal® | 16 | $5_{min}$ $10_{max}$ (gamme données fabricant) | 2h00 |
| 7 | Bactipal® | 16 | $5_{min}$ $10_{max}$ (gamme données fabricant) | 2h00 |
| 8 | acide peracétique (38%) | 16 | 5 | 2h00 |
| 9 | acide peracétique (38%) | 16 | 5 | 0h30 |
| 10 | acide peracétique (38%) | 8 | 2,5 | 1h10 |
| 11 | acide peracétique (38%) | 16 | 5 | 2h00 |
| 12 | acide peracétique (38%) | 16 | 5 | 2h00 |
| 13 | acide peracétique 38% | 16 | 5 | 2h00 |
| 14 | acide peracétique (38%) | 16 | 5 | 2h00 |
| 15 | acide peracétique (38%) | 16 | 5 | 2h00 |
| 16 | acide peracétique (38%) | 16 | 5 | 2h00 |
| 17 | $H_2O_2$ 30% | 8,6 | 7,8 | 10 minutes |
| 18 | $H_2O_2$ (30%) | 8,6 | 7,8 | 10 minutes |

**Tableau 3**

| Essai n° | Catalyseur d'oxydation | | |
|---|---|---|---|
| | Catalyseur | masse (g) | % ou ratio en poids cata/composition contenant du DFF |
| 1 | $RuCl_3$ | 0,068 | 1,7 |
| 2 | $RuCl_3$ | 0,068 | 1,7 |
| 3 | $KMnO_4$ (0.02 mol/L) | 2,53 | 0,2 |
| 4 | $KMnO_4$ (0.02 mol/L) | 4,03 | 0,63 |
| 5 | $KMnO_4$ (0.02 mol/L) | 1,26 | 0,2 |
| 6 | $KMnO_4$ (0.02 mol/L) | 4,03 | 0,63 |
| 7 | $KMnO_4$ (0.02 mol/L) | 4,03 | 0,63 |
| 8 | $KMnO_4$ (0.02 mol/L) | 4,03 | 0,63 |
| 9 | $KMnO_4$ (0.02 mol/L) | 1,2 | 0,2 |
| 10 | $KMnO_4$ (0.02 mol/L) | 0,4 | 0,063 |
| 11 | $KMnO_4$ (0.02 mol/L) | 0,4 | 0,063 |
| 12 | $K_2Cr_2O_7$ | 0,06 | 3 |
| 13 | $MoH_2O_4$ | 0,04 | 2 |
| 14 | $ZnCl_2$ | 0,035 | 1,75 |
| 15 | $FeCl_2$ | 0,035 | 1,75 |
| 16 | $FeCl_3$ | 0,045 | 2,25 |
| 17 | $FeCl_3$ | 0,11 | 9 |
| 18 | $Fe_2(SO_4)_3.5H_2O$ | 0,24 | 20 |

Le tableau 4 indique les temps de réaction et la température, ainsi que les teneurs en DFF, la sélectivité, et la conversion, relatifs aux réactions d'oxydation.

**Tableau 4**

| Essai n° | Température de réaction (°C) | Durée totale | Composition contenant du DFF | | Conversion (%) | Sélectivité en FDCA (%) |
|---|---|---|---|---|---|---|
| | | | Teneur en DFF (% poids) | Teneur en FFCA (% poids) | | |
| 1 | 50 | 6h00 | 99,3 | 0,7 | 100 | 91,7 |
| 2 | 60 | 3h30 | 99,3 | 0,7 | 100 | 75 |
| 3 | 50 | 5h00 | 95,1 | 4,9 | 100 | 91 |
| 4 | 50 | 5h00 | 73,3 | 26,7 | 100 | 87,2 |
| 5 | 50 | 5h00 | 73,3 | 26,7 | 100 | 84,7 |
| 6 | 50 | 5h00 | 73,3 | 26,7 | 100 | 80,5 |
| 7 | 30 | 5h00 | 73,3 | 26,7 | 100 | 66,2 |
| 8 | 40 | 5h00 | 73,3 | 26,7 | 100 | 77,5 |
| 9 | 50 | 0h45 | 93,1 | 6,9 | 100 | 72,6 |
| 10 | 50 | 1h10 | 93,1 | 6,9 | 100 | 70,7 |
| 11 | 50 | 2h15 | 93,1 | 6,9 | 100 | 80,5 |

(suite)

| Essai n° | Température de réaction (°C) | Durée totale | Composition contenant du DFF | | Conversion (%) | Sélectivité en FDCA (%) |
|---|---|---|---|---|---|---|
| | | | Teneur en DFF (% poids) | Teneur en FFCA (% poids) | | |
| 12 | 50 | 5h00 | 73,3 | 26,7 | 100 | 87,2 |
| 13 | 50 | 5h00 | 73,3 | 26,7 | 100 | 80,1 |
| 14 | 50 | 5h00 | 73,3 | 26,7 | 100 | 77 |
| 15 | 50 | 5h00 | 73,3 | 26,7 | 100 | 45 |
| 16 | 50 | 5h00 | 73,3 | 26,7 | 100 | 49.1 |
| 17 | 5 | 4h00 | 93,4 | 6,6 | 94 | 72* |
| 18 | 5 | 4h00 | 91,7 | 8,3 | 92 | 73* |

* pour les résultats des essais 17 et 18 la sélectivité correspond a la somme des sélectivités en fonctions carboxyliques et en fonctions esters méthyliques.

Les exemples, et notamment les valeurs obtenues selon le tableau 4, démontrent que le procédé selon la présente invention permet d'obtenir du FDCA avec un taux de conversion de 100 %. La sélectivité en FDCA est toujours supérieure à 70 %, et parfois même à 90 %.

On est donc parvenu à mettre au point un procédé facilement industrialisable de par sa simplicité et la disponibilité des réactifs utilisés, et de par les conditions expérimentales qu'il requiert : on travaille à pression atmosphérique et à de faibles températures (de l'ordre de 50 °C dans les exemples), les temps de réaction étant relativement courts (parfois inférieurs à 1 heure). De plus, on ne forme pas de sels qui nécessitent d'être traités en aval du procédé, et le ratio catalytique par rapport au produit initial, ratio en poids, est particulièrement faible. En réduisant la quantité de catalyseur engagée, on réduit d'autant le coût global du procédé, et on limite les risques liés à la dangerosité du catalyseur vis-à-vis des humains et de l'environnement.

## Revendications

**1.** Procédé de synthèse de l'acide 2,5-furane dicarboxylique (FDCA) à un pH inférieur à 4, par mise en contact :

a) d'une composition contenant du furane-2,5-dialdéhyde (DFF),
b) d'un solvant protique,
c) d'une source en oxydant,
d) et d'un catalyseur d'oxydation.

et **caractérisé en ce que** :

- le ratio entre catalyseur et DFF en poids est compris entre 0,01 % et 25 %, préférentiellement 0,01 et 10%, et plus préférentiellement entre 0,1 % et 3 % en poids ;
- la source en oxydant c) est l'acide peracétique introduit goutte à goutte ;
- le catalyseur d'oxydation d) est choisi $KMnO_4$ et,
- le solvant est choisi parmi l'eau, l'acide acétique et leurs mélanges.

**2.** Procédé selon la revendication 1, **caractérisé en ce que** la composition a) contenant du DFF est une mélange de DFF et acide 5-furaldéhyde-2-carboxylique (FFCA) en toutes proportions.

**3.** Procédé selon l'une des revendications 1 ou 2, **caractérisé en ce que** la composition contenant du DFF est issue de l'oxydation du 5-hydroxyméthyl furaldéhyde (5-HMF) en présence d'au moins un acide organique, d'un radical nitroxyl, d'une source en oxygène et d'un agent de transfert d'oxygène.

**4.** Procédé selon l'une quelconque des revendications 1 à 3, **caractérisé en ce que** la réaction d'oxydation a lieu à

une température comprise entre 3°C et la température de reflux du solvant, préférentiellement à une température comprise entre 40 °C et 60 °C.

**5.** Procédé selon l'une quelconque des revendications 1 à 4, **caractérisé en ce que** la pression correspond à la pression atmosphérique.

**6.** Procédé selon l'une quelconque des revendications 1 à 5, **caractérisé en ce que** le temps de contact entre les différents constituants a), b), c) et d) est compris entre 1 minute et 8 heures, préférentiellement entre 15 minutes et 2 heures.

**7.** Procédé selon l'une quelconque des revendications 1 à 6, **caractérisé en ce que** le nombre d'équivalent molaire en oxydant par rapport au DFF est compris entre 0,1 et 10, plus préférentiellement entre 2,5 et 5.

## Patentansprüche

**1.** Verfahren zur Synthese von 2,5-Furandicarboxylsäure (FDCA) bei einem pH unterhalb von 4 durch Inkontaktbringen:

a) einer Zusammensetzung enthaltend Furan-2,5-dialdehyd (DFF),
b) eines protischen Lösungsmittels,
c) einer Oxidationsmittelquelle,
d) eines Oxidationskatalysators,

und **dadurch gekennzeichnet, dass**:

- das Verhältnis zwischen dem Katalysator und dem DFF als Gewicht zwischen 0,01 Gew.-% und 25 Gew.-% umfasst ist, bevorzugt 0,01 und 10 Gew.-% und stärker bevorzugt zwischen 0,1 Gew.-% und 3 Gew.-%;
- die Oxidationsmittelquelle c) Peressigsäure ist, welche Tropfen für Tropfen hinzugefügt wird,
- der Oxidationskatalysator d) $KMnO_4$ gewählt wird, und
- das Lösungsmittel ausgewählt wird aus Wasser, Essigsäure und seinen Mischungen.

**2.** Verfahren gemäß Anspruch 1, **dadurch gekennzeichnet, dass** die Zusammensetzung a), welche DFF enthält, eine Mischung aus DFF und 5-Furaldehyd-2-carboxylsäure (FFCA) in allen Verhältnissen ist.

**3.** Verfahren gemäß Anspruch 1 oder 2, **dadurch gekennzeichnet, dass** die Zusammensetzung, welche DFF enthält, durch Oxidation von 5-Hydroxymethylfuraldehyd (5-HMF) in Anwesenheit von wenigstens einer organischen Säure, einem Nitroxylradikal, einer Sauerstoffquelle und einem Mittel zum Übertragen von Sauerstoff entstanden ist.

**4.** Verfahren gemäß einem der Ansprüche 1 bis 3, **dadurch gekennzeichnet, dass** die Oxidationsreaktion bei einer Temperatur umfasst zwischen 3 °C und der Refluxtemperatur des Lösungsmittels stattfindet, vorzugsweise bei einer Temperatur umfasst zwischen 40 °C und 60 °C.

**5.** Verfahren gemäß einem der Ansprüche 1 bis 4, **dadurch gekennzeichnet, dass** der Druck dem Atmosphärendruck entspricht.

**6.** Verfahren gemäß einem der Ansprüche 1 bis 5, **dadurch gekennzeichnet, dass** die Kontaktzeit zwischen den verschiedenen Bestandteilen a), b), c) und d) umfasst ist zwischen 1 Minute und 8 Stunden, vorzugsweise zwischen 15 Minuten und 2 Stunden.

**7.** Verfahren gemäß einem der Ansprüche 1 bis 6, **dadurch gekennzeichnet, dass** die Zahl der Moläquivalente an Oxidationsmittel gegenüber dem DFF zwischen 0,1 und 10 umfasst ist, stärker bevorzugt zwischen 2,5 und 5.

## Claims

**1.** A process for synthesizing 2,5-furandicarboxylic acid (FDCA) at a pH of less than 7, by bringing the following into contact:

a) a composition containing furan-2,5-dialdehyde (DFF),
b) a protic solvent,
c) a source of oxidizing agent,
d) and an oxidation catalyst,

and wherein
the weight ratio between catalyst and DFF is between 0.01% and 25%, preferentially between 0.01% and 10%, and more preferentially between 0.1% and 3% by weight,
the source of oxidizing agent c) is peracetic acid introduced dropwise,
the oxidation catalyst d) is $KMnO_4$,
the protic solvent b) is chosen from water and acetic acid, and mixtures thereof.

**2.** The process as claimed in claim 1, wherein the composition a) containing DFF is a mixture of DFF and 5-furaldehyde-2-carboxylic acid (FFCA) in any proportions.

**3.** The process as claimed in claim 1 or 2, wherein the composition containing DFF results from the oxidation of 5-hydroxymethyl furaldehyde (5-HMF) in the presence of at least one organic acid, of a nitroxyl radical, of an oxygen source and of an oxygen transfer agent.

**4.** The process as claimed in any one of claims 1 to 3, wherein the oxidation reaction is carried out at a temperature between 3°C and the reflux temperature of the solvent, preferentially at a temperature between 40°C and 60°C.

**5.** The process as claimed in any one of claims 1 to 4, wherein the pressure corresponds to atmospheric pressure.

**6.** The process as claimed in any one of claims 1 to 5, wherein the contact time between the various constituents a), b), c) and d) is between 1 minute and 8 hours, preferentially between 15 minutes and 2 hours.

**7.** The process as claimed in any one of claims 1 to 6, wherein the number of molar equivalents of oxidizing agent relative to DFF is between 0.1 and 10, more preferentially between 2.5 and 5.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2723945
- FR 2669634
- WO 2012017052 A
- US 20070232815 A
- FR 1162343


### Littérature non-brevet citée dans la description


- **WERPY, T. ; PETERSON, G.** *Top Value Added Chemicals from Biomass,* 2004, vol. 1, 26-28
- **ANDRISANO, R. ; ANGELONI, A.S.** *Ann. Chim. (Rome),* 1963, vol. 53, 1658
- Bulletin de la Société Chimique de France. *Soc. Fr. de Chim.,* 01 Janvier 1987, vol. 5, 855-860
- *Angewandte Chemie International Edition,* 23 Juin 2008, vol. 47 (27), 5038-5042
- **F. KOOPMAN ; N. WIERCKX ; J.H. DE WINDE ; H.J. RUIJSSENAARS.** *Proc. Nat. Acad. Sci. USA.,* 2010, vol. 107, 4919-4924